(19) Europäisches Patentamt
European Patent Office
Office européen des brevets

(11) **EP 3 191 444 B1**

(12) **EUROPEAN PATENT SPECIFICATION**

(45) Date of publication and mention of the grant of the patent:
**12.12.2018 Bulletin 2018/50**

(21) Application number: **15766775.9**

(22) Date of filing: **10.09.2015**

(51) Int Cl.:
**C07C 46/10** *(2006.01)*   **C07C 50/28** *(2006.01)*

(86) International application number:
**PCT/EP2015/070737**

(87) International publication number:
**WO 2016/038146 (17.03.2016 Gazette 2016/11)**

(54) **RECRYSTALLIZATION OF UBIDECARENONE FOR IMPROVED BIOAVAILABILITY**

NEUKRISTALLISATION VON UBIDECARENON ZUR VERBESSERTEN BIOVERFÜGBARKEIT

RECRISTALLISATION DE L'UBIDÉCARÉNONE PERMETTANT UNE MEILLEURE BIODISPONIBILITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2014 EP 14184327**
**10.09.2014 EP 14184328**

(43) Date of publication of application:
**19.07.2017 Bulletin 2017/29**

(73) Proprietor: **Pharma Nord ApS**
**6500 Vojens (DK)**

(72) Inventors:
• **MOESGAARD, Sven**
**DK-6051 Almind (DK)**

• **PAULIN, Helge, Schousen**
**DK-6580 Vamdrup (DK)**

(74) Representative: **COPA Copenhagen Patents**
**Rosenørns Allé 1, 2nd floor**
**1970 Frederiksberg C (DK)**

(56) References cited:
**CN-A- 103 819 326     US-A- 4 220 719**

• **DATABASE CA [Online] CHEMICAL ABSTRACTS SERVICE, COLUMBUS, OHIO, US; "Coenzyme Q10", XP002751091, retrieved from STN Database accession no. 1982:18660 & JP S56 106597 A (MITSUBISHI GAS CHEMICAL CO., INC., JAPAN) 24 August 1981 (1981-08-24)**

EP 3 191 444 B1

**EP 3 191 444 B1**

**Description**

FIELD OF THE INVENTION

The present invention relates to a crystal form of ubidecarenone, wherein crystals are non-spherical and wherein the ratio of the crystal surface area per volume - calculated as $\mu m^2/\mu m^3$ - of ubidecarenone is greater than 2, and wherein the crystal form of ubidecarenone comprises a plurality of needle-shaped or cone-shaped elongated parts have an average length of at least 10 $\mu m$ and a diameter at the base of less than 1 $\mu m$, said elongated parts extending or protruding from a core region.

The invention further relates to a method for producing such crystal form of ubidecarenone as well as to compositions comprising ubidecarenone.

BACKGROUND OF THE INVENTION

Ubidecarenone (also known as coenzyme Q10 (CoQ10) or ubiquinone), is a 1,4-benzoquinone, where Q refers to the quinone chemical group, and 10 refers to the number of isoprenyl chemical subunits in its tail.

Ubidecarenone is present in most eukaryotic cells, primarily in the mitochondria. It is a component of the electron transport chain and participates in aerobic cellular respiration, generating energy in the form of ATP. There are three redox states of CoQ10: fully oxidized (ubiquinone), semiquinone (ubisemiquinone), and fully reduced (ubiquinol). The capacity of this molecule to exist in a completely oxidized form and a completely reduced form enables it to perform its functions in the electron transport chain, and as an antioxidant, respectively. The highest ubidecarenone concentrations are found in heart, liver and kidney.

Ubidecarenone is contained in food and is also endogenously produced in the body. It works both as an antioxidant and as an energy transporter in the inner membrane of mitochondria. As a medicine, it is used for heart failure patients, and as a food supplement, it is consumed for nutritional purposes.

Ubidecarenone is a highly lipophilic substance that is practically insoluble in water. It is a large molecule (863 g/mol) which has low bioavailability when ingested. It is soluble in ethanol, hexane and fats or oils. The melting point of Q10 is 48°C. Exposure of Q10 to higher temperatures for longer time turns ubidecarenone into ubichrominol-9 or other undesired degradation products.

Ubidecarenone is manufactured in bulk as a powder consisting of rhomboid crystals. The applicant has analysed Q10 crystals from a range of producers by microscopy. The exact size (but not the shape/form) of the crystals may vary due to different degrees of grinding. However, in general such bulk API materials are flattened crystals being approximately 50 x 50 $\mu m$ in length and width (with a range of from approximately 10 x 10 $\mu m$ to 200 x 200 $\mu m$), and having a height of approximately 3 $\mu m$.

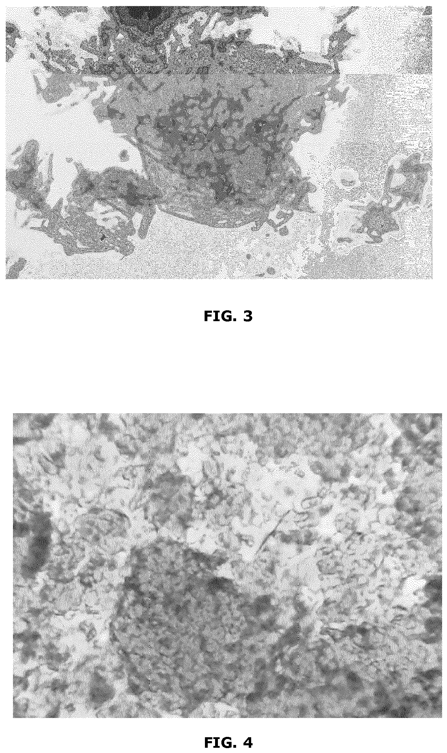

Pictures of such crystals are shown in figures 3 and 4.

CN 103819832 A describes a process for separation and purification of Coenzyme Q10 from microorganisms. In order to produce Coenzyme Q10 in high concentration seed crystals are added for induction of crystallization, followed by washing with methanol and water and subsequent drying. A similar method is disclosed in CN103819326 A. In neither document is there an indication of any unknown or surprising crystal structure of the obtained product.

US 4,220,719 A1 describes a process for production of concentrated Coenzyme Q10 from selected microorganisms. A series of purification steps are applied in order to obtain a high concentration of Coenzyme Q10. Crystals of Coenzyme Q10 are formed and recrystallized by use of ethyl alcohol. No specification of the crystal structure of the final product is included, and there is no indication as to any unknown or surprising crystal structure of the product that is obtained.

Japanese patent application JPS516106597 A discloses the isolation of ubiquinone in the form of plate-like crystals.

These references do not disclose methods leading to the crystal Coenzyme Q10 according to the present invention, which are formed in the presence of particular fat/oil compositions and under specific temperature-dependent controlled conditions (se examples and reference examples in the experimental section).

Powder of ubidecarenone can be dissolved in heated fat or oil. Conventional compositions of Q10 for human consumption therefore usually comprise fat or oil in which Q10 is solubilized or partly solubilized depending on the concentration of Q10 and the temperature.

Thus, softgel capsules comprising ubidecarenone and fat or oil are well known in the art.

Upon cooling to below its melting point (in the respective fat or oil), oversaturated solutions of Q10 in traditional fat or oil compositions spontaneously form rhomboid crystals typically 50 x 50 $\mu m$ and varying from 10 - 200 $\mu m$. The ubidecarenone present in these compositions is in a form that impairs the bioavailability of Q10. Traditional softgel formulations consist of 10 - 200 mg ubidecarenone and 300 - 1000 mg fat or oil in a softgel capsule. The majority of Q10 present in such capsules is present as rhomboid crystals. See in this respect the reference example 3 in the present

2

specification.

Accordingly, the delivery of Q10 is impaired by the very low solubility in water and the resulting low dissolution rate after ingestion of the capsules containing mainly rhomboid crystals of ubidecarenone. Consequently, Q10 poses significant problems in terms of delivery to the human or animal body. Undissolved Q10 will not enter the human or animal body where it exerts its beneficial effects.

Several possibilities for increasing the dissolution rate of Q10 *in vivo* has been explored, most of which focus on providing delivery of Q10 as micronized particles.

However, the substantially spherical particles resulting from micronization are undesirable as they are difficult to work with and tend to reaggregate during formulation and storage. Further, solubilizing micronized Q10 in fat or oil will result in re-appearance of rhomboid crystals when Q10 recrystallizes in the formulations during storage.

Thus, there is a need in the art for new compositions or crystal forms of Q10 providing increased dissolution *in vivo,* and thereby providing improved bioavailability when ingested by human or animal subjects, as well as manufacturing methods for the production of such.

Further, there is a need in the art for improved compositions comprising Q10.

The crystal formation of Q10 in softgel capsules can be observed by microscope, measured by nuclear magnetic resonance (NMR) or directly measured after separating the content of a capsule by high gravity centrifugation. Further, the bioavailability of ubidecarenone can be measured by supplementing humans in controlled trials with different types of capsules. In vitro testing of release profiles with standard methods may further simulate bioavailability.

## SUMMARY OF THE INVENTION

During experiments leading to the present invention, it was surprisingly found that new crystal forms of ubidecarenone comprising a plurality of elongated parts, the elongated parts extending or protruding from a core region, could be produced. The Q10 crystal form was characterised in having a visual appearance as an assembly of spikes growing outwards from a central region. Another appropriate description is that the crystals in their three-dimensional structure are star-shaped. The surface-to-volume-ratios of these new crystals were observed to be comparable or even higher than previously known micronized Q10 particles.

This finding made possible the production of compositions comprising discrete particles of Q10, each particle providing a high bioavailability. These compositions provide improved bioavailability of ubidecarenone. Further, the compositions provide extended shelf-life of ubidecarenone in bioavailable form.

Accordingly, the invention relates to a crystal form of ubidecarenone, wherein the ratio of the crystal surface area per volume - measured in $\mu m^2/\mu m^3$ - of ubidecarenone is greater than 2 and the crystal form of ubidecarenone comprises a plurality of needle-shaped or cone-shaped elongated parts having an average length of at least 10 $\mu m$ and a diameter at the base of less than 1$\mu m$, said elongated parts extending or protruding from a core region.

The new crystal form of ubidecarenone was surprisingly obtainable by use of the method described in the experimental part of the present application.

Essentially, the inventive method comprises the steps of solubilizing powdered Q10 particles into a composition comprising fat or oil having a low melting point (e.g. a low temperature melting triglyceride having a melting temperature of below 15°C) and oil having a high melting point (e.g. a high-temperature melting triglycerides having a melting temperature of above 30°C), followed by cooling of the resulting compositions, preferably to a temperature below the melting point of Q10 (the melting point in the respective composition), even more preferably to a temperature below 37°C. It was found essential that the melting temperature of the different oil constituents (high vs. low temperature melting) differed by at least 10 °C.

Accordingly, in one embodiment the present invention relates to a composition comprising ubidecarenone, one or more low-temperature melting triglycerides, and one or more high-temperature melting triglycerides, said low-temperature melting triglycerides having a melting point that is at least 10°C lower than the melting temperature of the high-temperature melting triglycerides.

In a highly preferred embodiment, the low-temperature melting triglyceride has a melting point below 15°C.

In a highly preferred embodiment, the high-temperature melting triglyceride has a melting point that exceeds 30°C.

Accordingly, in a preferred embodiment, the present invention relates to a composition comprising ubidecarenone, one or more low-temperature melting triglycerides, and one or more high-temperature melting triglycerides, said low-temperature melting triglycerides having melting points below 15°C, and said high-temperature melting triglycerides having melting points that exceed 30°C.

The invention further relates to a method of producing the new crystal forms of Q10, the method comprising the steps of:

a. providing fat or oil composition comprising one or more low-temperature melting triglycerides and one or more high-temperature melting triglycerides, said low temperature melting triglycerides having melting points at least 10°C lower than the melting temperature of the high-temperature melting triglycerides,

b. solubilizing ubidecarenone in the composition of step a),

c. lowering the temperature of the composition of step b) to a temperature below 40°C, thereby providing an oversaturated composition, for a period sufficient to allow formation of the Q10 crystals according to the invention, and

d. optionally, cooling the composition of step c) to room temperature.

More preferably, the inventive method of producing the new crystal forms of Q10 comprises the steps of:

a) providing fat or oil composition comprising one or more low-temperature melting triglycerides and one or more high-temperature melting triglycerides, said low-temperature melting triglycerides having melting points that are at least 10°C lower than the melting temperature of the high-temperature melting triglycerides,

b) solubilizing ubidecarenone in the composition of step a),

c) lowering the temperature of the composition of step b) to a temperature below the temperature at which the amount of Q10 solubilizes in the composition, thereby providing an oversaturated composition, for a period sufficient to allow formation of Q10 crystals according to claim 1, and

d) optionally, cooling the composition of step c) to room temperature.

Preferably, the composition of step a) is heated to a temperature that is above the melting temperature of the one or more high-temperature melting triglycerides.

Further, it is preferred that the composition of step b) is saturated or nearly saturated with ubidecarenone.

Preferably, in step a), the low temperature-melting triglycerides have a melting point of 15°C or below. Further, it is preferred that the high-temperature melting triglycerides have a melting point of 30°C or above.

Step b) is preferably performed by heating the composition of step a) to a temperature of at least 47°C followed by cooling and maintaining the composition at a temperature of between 40°C and 47°C, preferably 43°C, for a period of time sufficient to ensure complete solubilization of Q10.

Preferably, the addition of ubidecarenone is performed immediately after heating the composition of step a) to a temperature of at least 47°C. Alternatively, the addition of ubidecarenone is performed prior to the heating of the composition of step a) to a temperature of at least 47°C.

The compositions according to the invention may be filled into softgel capsules, e.g. as part of the optional step d) of the above process. Filling the compositions into softgel capsules may be accomplished by use of standard equipment.

The invention further relates to softgel capsules comprising the new crystal form of Q10.

DEFINITIONS

"High-temperature-melting fat or oil" according to the invention means, in its broadest sense, a triglyceride that has a melting point (temperature) that is at least 10°C higher than the melting point (temperature) of the low-temperature melting fats or oils. Preferably, however, "High-temperature-melting fat or oil" according to the invention has a melting point (temperature) of at least 30°C, as determined by appropriate methods described in Ph.Eur. Alternative methods are described in DAB (deutsches Arzneibuch) or British Pharmacopoeia. The "high-temperature melting fat or oil" according to the invention may be a natural triglyceride or a triglyceride which has been hydrogenated or partly hydrogenated to increase the melting temperature. Alternatively, it may be a natural hydrogenated or partly hydrogenated triglyceride or a natural non-hydrogenated triglyceride, preferably, with a melting point of at least 30°C.

"Low-temperature melting fat or oil" according to the invention means, in its broadest sense, a triglyceride that has a melting point (temperature) that is at least 10°C lower than the melting point (temperature) of the high temperature melting fats or oils according to the invention. Preferably, however, "Low-temperature-melting fat or oil" according to the invention means a triglyceride with a melting point below 15°C determined by methods described in Ph.Eur. Alternative methods are described in DAB (deutsches Arzneibuch) or British Pharmacopoeia.

Unless otherwise stated, parts, concentrations or ratios mentioned in the present description are based on weight (w/w).

"Surface to volume ratio" according to the invention mean the surface area measured in $\mu m^2$ divided with the volume measured in $\mu m^3$. As an example of such calculation, a cube with a length, with and height of 1 $\mu m$ will have a "surface to volume ratio" according to the invention of 6 (surface area of 6 $\mu m^2$ and a volume of 1 $\mu m^3$). Importantly, the "surface to volume ratio" of the same cube (1 $\mu m$ x 1 $\mu m$ x 1 $\mu m$), but determined as the surface area measured in $mm^2$ divided with the volume measured in $mm^3$ would give 6000 (0.000006 divided by 0.000000001).

**Brief description of figures**

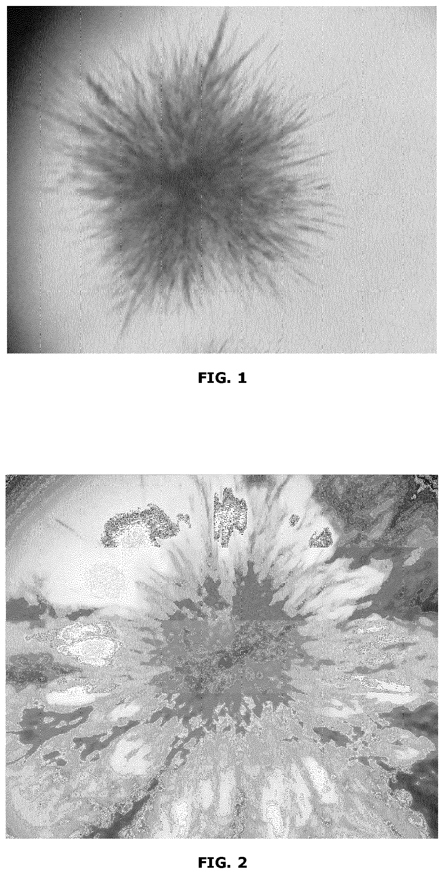

Figure 1 show a microscopic picture (in 1000x magnification, each side being approximately 100 $\mu$m) of the crystal form of ubidecarenone according to the invention. The crystal is compressed under the glass of the microscope. As can be seen, the crystal (in its 3-dimensional form) is star-shaped and comprises a plurality of needle-shaped or cone-shaped elongated parts have an average length of at least 10 $\mu$m and a diameter at the base of less than 1$\mu$m, said elongated parts extending or protruding from a core region.

Figure 2 show a microscopic picture (in 1000x magnification, each side being approximately 100 $\mu$m) of the crystal form of ubidecarenone according to the invention. The crystal is compressed under the glass of the microscope. As can be seen, the crystal (in its 3-dimensional form) is star-shaped and comprises a plurality of needle-shaped or cone-shaped elongated parts have an average length of at least 10 $\mu$m and a diameter at the base of less than 1$\mu$m, said elongated parts extending or protruding from a core region. The elongated parts of the crystal shown comprise further crystal growth at the ends of the elongated parts.

Figure 3 and 4 show a microscopic picture (in 250x magnification, the sides being approximately 500 $\mu$m and 600 $\mu$m, respectively) of the raw API crystal form of ubidecarenone. As can be seen, the API crystal is substantially rhomboid.

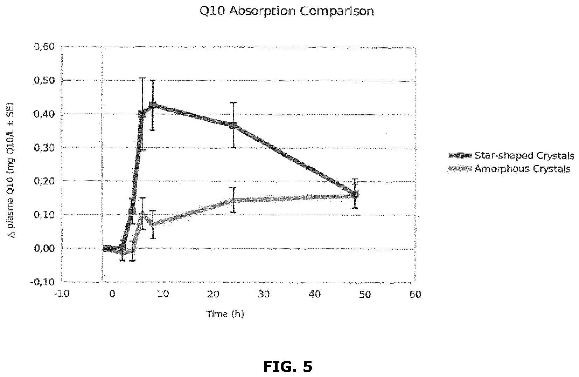

Figure 5 show the result of the experiment performed in example 8, i.e. the plasma concentration above baseline concentration (mean$\pm$SE) of Q10 during the first 48 hours in the subject treated with the compositions comprising the product (POW597) containing star-shaped crystals according to the invention (top curve) vs. the concentration (mean$\pm$SE) of ubidecarenone in plasma in the subject treated with the compositions comprising the reference product (KOJ786).

DETAILED DESCRIPTION OF THE INVENTION

During the development work, a new type of crystals of ubidecarenone has been produced.

The new crystal form of ubidecarenone has improved properties in that it provides an increased dissolution rate in water and improved bioavailability *in vivo.*

Further, the crystals, once formed, can be maintained in stable form in compositions according to the invention, said compositions comprising one or more low-temperature melting triglycerides, and one or more high-temperature melting triglycerides, even after re-heating and subsequent cooling.

The new form of ubidecarenone is produced by melting ubidecarenone powder in a characteristic fat/oil matrix and mixing the ingredients according to a special procedure at controlled temperatures and time intervals.

In a preferred embodiment, the fat or oil composition used according to the invention to initially dissolve the ubidecarenone powder, consists of a fat/oil matrix comprising a natural or hydrogenated triglyceride that contains saturated fatty acids with high melting points, and a natural or non-hydrogenated triglyceride that contains unsaturated fatty acids with low melting points.

As seen in the below examples, *in vitro* tests in form of dissolution studies show that the new needle-shaped ubidecarenone crystals according to this invention dissolve more rapidly and completely at human body temperature of 37°C than the conventional rhomboid crystals.

*In vitro* solubility tests have shown that at the ratio 100 mg ubidecarenone to 400 mg oil matrix (which corresponds to the formulations of the examples) all ubidecarenone is in a dissolved form at 37 °C after 10 minutes, if the ubidecarenone is manufactured according to the invention. Thus, the needle-shaped crystals will dissolve in the capsule matrix of the examples within 10 - 15 minutes at 37°C, which is faster than the *in vitro* disintegration expected for soft gelatin capsules. Accordingly, the rate-limiting step in the drug release will be expected to be the disintegration of the capsules. *In vivo* release of the content of the soft gelatin capsules according to the invention, will probably take place at a somewhat slower rate ensuring that all ubidecarenone is dissolved in the capsule matrix before it is released in the GI tract after oral intake. Therefore, the ubidecarenone crystals according to the invention are released as a solution into the GI tract, rather than as a mixture of dissolved drug substance and crystals.

A high portion of needle-shaped (star-shaped) crystals ($\geq$ 99%) according to the present invention is considered preferable, which is ensured by carefully solubilizing all rhomboid ubidecarenone API in the formulations prior to initiating the formation of the crystals according to the invention.

The ubidecarenone crystals according to the invention appear in microscope as star-shaped crystals comprising multiple needle-shaped cylinders with average lengths of approximately 10 $\mu$m - 100 $\mu$m and diameters of less than 1

μm protruding from a central core.

The amount of ubidecarenone in solution is dependent on the temperature. At room temperature (20°C), as much as 80% of ubidecarenone in a softgel capsule according to the invention may be found to be in crystals, such as stars or needle-shaped crystals according to the invention. Large rhomboid crystals are not found in the compositions according to the invention.

As seen in the experimental section, *in vitro* and *in vivo* experiments have documented that ubidecarenone from softgel capsules according to the invention is better absorbed by the human body than ubidecarenone from softgel capsules that contain rhomboid crystals.

New crystal form according to the invention

The ubidecarenone crystals produced according to the invention in the fat or oil matrix as described, crystallizes into needle-shaped crystals when cooled (to below melting temperature of Q10). These crystals appeared as multiple needle-shaped cylinders with lengths from 2 to 12 μm and diameters of approximately 1 μm protruding from a central core region.

These crystals also appeared as multiple needle-shaped cylinders with average lengths from 10 to 100 μm and diameters of less than 1 μm protruding from a central core region, i.e. as multiple needle-shaped cylinders with average lengths from 10 to 100 μm and diameters of 0.01 - 1 μm protruding from a central core region.

The new crystal form of Q10 was characteristic in having a tremendously increased crystal surface area (the surface area compared to the volume of the crystal structure).

As the needle-shaped crystals (bundles of needles) according to this invention are essentially cylindrical, the crystal surface area to volume could be estimated (under-estimated) by reference to the surface area (A) and volume (V) of a cylinder 10 μm long and 1 μm in diameter:

$$A = 2 \times \pi \times 0.5\ \mu m \times 10\ \mu m + 2 \times \pi \times (0.5\ \mu m)^2 = 33.0\ \mu m^2$$

$$V = \pi \times (0.5\ \mu m)^2 \times 10\ \mu m = 7.9\ \mu m^3$$

The crystal surface area per 1 μm³ needle-shaped ubidecarenone is thereby estimated (under-estimated) to be 33.0/7.9 = 4.2.

An even more appropriate estimation of the crystal surface area to volume ratio of the Q10 particles of the invention, however, could be to base the estimate on the surface/volume of a corresponding cone-shaped structure (calculated excluding surface area of base) whereby the crystal surface area per volume would be estimated to be around 6.

However, even this estimation is an underestimation, as the average diameter of the elongated parts of the crystals according to the invention is estimated to be less than 0.5 μm in diameter at the base.

Thus, even more specifically, the needle-shaped crystals according to this invention are essentially cylindrical (bundles of needles), with an average length of approximately 10 μm and a diameter at the base of less than 0.5 μm, thus having an approximate surface to volume ratio of a 10 μm long cylinder being 0.5 μm in diameter:

$$A = 2 \times \pi \times 0.25\ \mu m \times 10\ \mu m + 2 \times \pi \times (0.25\ \mu m)^2 = 16\ \mu m^2$$

$$V = \pi \times (0.25\ \mu m)^2 \times 10\ \mu m = 2\ \mu m^3$$

The crystal surface area per 1 μm³ needle-shaped ubidecarenone is thereby estimated to be 16/2 = 8.

An even more appropriate estimation of the crystal surface area of the Q10 particles of the invention, however, could be to base the estimate on the surface/volume of a corresponding cone-shaped structure (calculated excluding surface area of base) whereby the crystal surface area per volume would be estimated to be around more than 10.

In contrast hereto, reference rhomboid crystals appearing in conventional Q10 compositions and powders are typically ∼ 50 x 50 μm (varying from approximately 10 x 10 μm to 200 x 200 μm) with a height of approximately 3 μm.

These rhomboid crystals are essentially flattened squares, and their surface area and volume can be compared to that of a box with dimensions of 50 μm x 50 μm x 3 μm. The area (A) and volume (V) of such a crystal can be calculated to be:

$$A = 2 \times 50 \ \mu m \times 50 \ \mu m + 4 \times 50 \ \mu m \times 3 \ \mu m = 5,600 \ \mu m^2$$

$$V = 3 \ \mu m \times 50 \ \mu m \times 50 \ \mu m = 7,500 \ \mu m^3$$

The crystal surface area per 1 $\mu m^3$ rhomboid ubidecarenone is thereby estimated to be 5600/7500 = 0.75.

Thus, needle-shaped ubidecarenone crystals have a surface area to volume ratio that is at the very least 5.6 (4.2 : 0.75= 5.6), but most probably around 8 (6.0 : 0.75) times greater than that of rhomboid crystals.

In further comparison hereto, micronized crystals of Q10 (estimated median particle size and shape: Spherical crystal, radius: 1-2$\mu m$) have an estimated crystal surface area ($\mu m^2$) per volume ($\mu m^3$) of between 1.5 (radius 2) and 3 (radius 1).

Thus, the new crystal form of the present invention provides a markedly increased crystal surface area.

Thus, the invention relates to a crystal form of ubidecarenone, wherein the crystal form of ubidecarenone has a non-spherical, non-rhomboid physical form. The inventive crystal form is obtained without micronisation. Accordingly, the invention relates to a non-micronized crystal form of ubidecarenone, wherein the crystal form of ubidecarenone has a non-spherical, non-rhomboid physical form.

The invention relates to a crystal form of ubidecarenone, wherein the crystal form comprises a plurality of elongated parts, the elongated parts extending or protruding from a core region. The invention further relates to a crystal form of ubidecarenone, wherein the elongated parts are needle shaped or cone shaped. The invention further relates to a crystal form of ubidecarenone, wherein the elongated parts are needle-shaped or cone-shaped and have an average length of at least 10 $\mu m$ and a diameter at the base of less than 1$\mu m$. The invention further relates to a crystal form of ubidecarenone, wherein the elongated parts are needle-shaped or cone-shaped and have an average length of between 10 and 100 $\mu m$ and an average diameter at the base of between 0.001 - 1$\mu m$.

The invention further relates to a crystal form of ubidecarenone, wherein the ratio of the crystal surface area per volume - measured as $\mu m^2/\mu m^3$ - of ubidecarenone is greater than 2.

In another embodiment, the invention relates to a crystal form of ubidecarenone, wherein the ratio of the crystal surface area per volume - measured as $\mu m^2/\mu m^3$ - of ubidecarenone is greater than 3.

In another embodiment, the invention relates to a crystal form of ubidecarenone, wherein the ratio of the crystal surface area per volume - measured as $\mu m^2/\mu m^3$ - of ubidecarenone is greater than 4.

In another embodiment, the invention relates to a crystal form of ubidecarenone, wherein the ratio of the crystal surface area per volume - measured as $\mu m^2/\mu m^3$ - of ubidecarenone is greater than 5.

In another embodiment, the invention relates to a crystal form of ubidecarenone, wherein the ratio of the crystal surface area per volume - measured as $\mu m^2/\mu m^3$ - of ubidecarenone is between 5 and 7.

In another embodiment, the invention relates to a crystal form of ubidecarenone, wherein the ratio of the crystal surface area per volume - measured as $\mu m^2/\mu m^3$ - of ubidecarenone is greater than 6.

In another embodiment, the invention relates to a crystal form of ubidecarenone, wherein the ratio of the crystal surface area per volume - measured as $\mu m^2/\mu m^3$ - of ubidecarenone is greater than 7.

In another embodiment, the invention relates to a crystal form of ubidecarenone, wherein the ratio of the crystal surface area per volume - measured as $\mu m^2/\mu m^3$ - of ubidecarenone is greater than 8.

In another embodiment, the invention relates to a crystal form of ubidecarenone, wherein the ratio of the crystal surface area per volume - measured as $\mu m^2/\mu m^3$ - of ubidecarenone is between 6 and 10, or even between 6 and 100.

In one embodiment, the crystal form of ubidecarenone according to the present invention is intended for use as a medicine.

In another embodiment, the invention relates to the use of the crystal form of ubidecarenone according to the present invention as a food or food supplement.

*Compositions according to the invention*

The compositions according to the invention comprise needle-shaped ubidecarenone, and at least one excipient, preferably fat or oil wherein ubidecarenone is dispersed.

In a preferred embodiment, the compositions according to the present invention comprise ubidecarenone, at least one low-temperature melting triglyceride, and at least one high-temperature melting triglyceride, said low-temperature melting triglyceride having a melting point that is at least 10°C lower than the melting temperature of the high-temperature melting triglycerides. These compositions are characterised in containing dispersed ubidecarenone crystals at room temperature (20°), and solubilized ubidecarenone at a temperature of (37°) or above.

In a highly preferred embodiment, the low-temperature melting triglyceride has a melting point below 15°C. In a preferred embodiment, the low temperature melting triglyceride is a naturally occurring fat or oil. Examples of preferred naturally-occurring low-temperature melting triglycerides are almond oil, canola oil, corn oil, cottonseed oil, flaxseed oil, grape seed oil, hemp seed oil, olive oil, peanut oil, rapeseed oil, rice bran oil, safflower oil, sesame oil, soybean oil and sunflower oil.

In a preferred embodiment, the high-temperature melting triglyceride has a melting point at or around 25°C. In a preferred embodiment, the high-temperature melting triglyceride is a naturally occurring fat or oil. Examples of such preferred naturally occurring high- temperature melting triglycerides are palm kernel and coconut oil (melting temperature around 25°C). In a highly preferred embodiment, the high-temperature melting triglyceride have a melting point that exceeds 30°C.

Examples of such highly preferred naturally occurring high-temperature melting triglycerides are palm oil, shea butter and cocoa butter. Other highly preferred high-temperature melting triglycerides are naturally occurring triglycerides that have been hydrogenated, such as hydrogenated soybean oil.

Accordingly, in a preferred embodiment, the present invention relates to a composition comprising ubidecarenone, one or more low-temperature melting triglycerides, and one or more high-temperature melting triglycerides, said low-temperature melting triglycerides having melting points below 15°C, and said high-temperature melting triglycerides having melting points that exceed 30°C.

In an even more preferred embodiment, the present invention relates to a composition comprising ubidecarenone, one or more low-temperature melting triglycerides, and one or more high-temperature melting triglycerides, said low-temperature melting triglycerides being naturally occurring triglycerides having melting points below 15°C, and said high-temperature melting triglycerides being a naturally occurring triglyceride having melting points that exceed 30°C.

Preferably, the one or more low-temperature melting triglycerides comprise the bulk of the triglyceride matrix in the compositions, said low-temperature melting triglycerides being present in excess of the high-temperature melting triglycerides. A preferred ratio between low- and high-temperature melting fats and oils was found to be between 2:1 - 100:1.

Further, as part of the present invention, research has been performed in order to find the minimum capsule size (the minimal composition) that may contain the largest amount of needle-shaped crystals of ubidecarenone and still go quickly into solution *in vivo* at a temperature of 37°C *in vivo.* The optimal ratio was found to be approximately 1:4 between ubidecarenone and triglyceride matrix.

Accordingly, in a preferred embodiment, the compositions according to the invention comprise 1 part Q10 per 2 - 20 parts fat or oil. Even more preferred, the compositions according to the invention comprise 1 part Q10 per 2 - 6 parts fat or oil. Even more preferred, the compositions according to the invention comprise 1 part Q10 per 3 - 5 parts fat or oil. Even more preferred, the compositions according to the invention comprise 1 part Q10 per 4 parts fat or oil.

Thus, the invention relates to a composition, wherein the ratio of fat and/or oil to ubidecarenone per weight is between 2:1 and 20:1, preferably 2:1 and 6:1, preferably 4:1.

In a highly preferred embodiment, the composition according to the invention comprises soybean oil as the low-temperature melting fat or oil, and hydrogenated soybean oil with a melting point of above 30°C as the high-temperature melting fat(s) or oil(s). In a preferred embodiment, the ratio between soybean oil and hydrogenated soybean oil is around 3:1.

The compositions according to the invention may also contain further ingredients. An appropriate example of further ingredients is antioxidants, such as e.g. vitamin E.

A highly preferred composition according to the invention is a softgel capsule containing a composition according to the invention, the composition comprising (per 500 mg composition) approximately 100 mg ubidecarenone, 297 mg soybean oil, 79 mg hydrogenated soybean oil with a melting point of 32°C, 20 mg hydrogenated soybean oil with a melting point of 41°C, and 4 mg Vitamin E 1 IU/mg.

A typical softgel capsule shell is composed of gelatine, glycerol, water and colouring agents.

In one embodiment, the compositions according to the present invention are intended for use as a medicine.

In another embodiment, the invention relates to the use of the compositions according to the present invention as a food or food supplement.

*Process according to the invention*

The invention further relates to a method of producing the new crystal forms of Q10, the method comprising the steps of:

> a. providing a fat or oil composition comprising one or more low-temperature melting triglycerides and one or more high-temperature melting triglycerides, said low-temperature melting triglycerides having melting points at least 10°C lower than the melting temperature of the high-temperature melting triglycerides,
> b. solubilizing ubidecarenone in the composition of step a),

c. lowering the temperature of the composition of step b) to a temperature below the temperature at which Q10 solubilizes in the composition, thereby providing an oversaturated composition, for a period sufficient to allow formation of the Q10 crystals according to the invention, and
d. optionally, cooling the composition of step c) to room temperature.

It was found that the production of crystals according to the invention was improved in manufacturing processes that include the following detailed steps:

Step a):

The composition of step a) should preferably be provided in fluent form (e.g. at a temperature of above the melting temperature of (each of) the high-temperature melting triglycerides. The composition must be fluid if heated to 47°C.

Preferably, said low-temperature melting triglycerides have melting points below 15°C and said high-temperature melting triglycerides have melting points of at least 30°C.

Further, it is preferred that at least one of the high-temperature melting triglycerides used in step a) are selected among the group of fats and/or oils comprising hydrogenated or partially hydrogenated triglycerides as well as natural fat or oils with a high concentration of saturated fat. Highly preferred fat/oils are cocoa butter, coconut oil, hydrogenated soybean oil, palm kernel oil, palm oil or shea fat/oil.

Preferably, the high-temperature melting triglycerides used are natural triglycerides, such as cocoa butter, coconut oil, palm kernel oil, palm oil or shea fat.

Further, it is preferred that at least one of the low temperature melting triglycerides used in step a) are selected among the group of fats and/or oils comprising a low amount of saturated fat such as almond oil, canola oil, corn oil, cottonseed oil, flaxseed oil, grape seed oil, hemp seed oil, olive oil, peanut oil, rapeseed oil, rice bran oil, safflower oil, sesame oil, soybean oil and sunflower oil.

The oil/fat matrix provided in step a) is preferably produced by heating the low-temperature melting oil to a temperature of above the melting point of the high-temperature melting triglyceride to be used.

The high-temperature melting fat/oil is then melted into the low-temperature melting fat/oil providing a homogeneous matrix.

Step b):

Step b) is preferably performed by heating the composition of step a) to a temperature of at least 47°C. Thus, a preferred temperature at which to add Q10 was found to be 47°C. Preferably the addition of ubidecarenone is performed immediately after the heating the composition of step a) to a temperature of at least 47°C, whereby the degradation of ubidecarenone is limited as much as possible. Alternatively, the addition of ubidecarenone is performed prior to the heating of the composition of step a) to a temperature of at least 47°C.

The homogeneous matrix comprising the high- and the low-temperature melting fat/oil is thereafter maintained at a temperature at which Q10 will solubilize completely in the composition. However, it is preferred to keep the solubilizing temperature as low as possible in order to avoid degradation of Q10. It is highly preferred that all Q10 is solubilized, as even a minimal amount of remaining (unsolubilized) Q10 material as rhomboid crystals will reduce or even preclude the formation of crystals according to the invention in step c) of the process. It was found preferable to solubilize Q10 at a temperature below the melting point of Q10. However, lowering the temperature of the composition too fast and/or too much will decrease the formation of needle-shaped crystals according to the invention (in step c) probably due to presence of undissolved rhomboid crystals of Q10. On the other hand, high temperatures or longer processing times tend to give more degradation of ubidecarenone in the fat or oil (triglyceride) matrix. Thus, there is a risk that keeping the compositions at elevated temperatures for longer periods of time will increase the degradation of Q10.

Preferable results were obtained by cooling and maintaining the composition in step b) at a temperature between 40°C and 47°C, preferably 43°C, for a period of time between 30 minutes and 10 hours.

Keeping the compositions in step b) of the process at a temperature of 43°C for 1 hour yielded more than 99% crystals according to the invention (documented by use of pulse NMR analyses and by saturation measurements) in the following step c).

Preferably, the composition of step b) is saturated with ubidecarenone prior to the performance of step c)

Step c):

After completing the mixing and solubilizing of the added Q10, the temperature of the compositions is lowered to below the solubilization temperature of Q10 in the composition. This exact solubilisation temperature of Q10 in the composition may be dependent on the characteristics of the particular fat/oil mixture in the composition. However, preferably, the temperature of the compositions is lowered to below 40°C, even more preferably below 37°C, even more preferably below 35°C, such as below 33°C, or below 30°C, whereby an oversaturated composition is provided. Thereby, Q10 spontaneously crystallizes as needle-shaped crystals according to the invention. It was observed (by visual inspection) that small points of crystallisation (seeds) start to form in the initial cooling phase of step c). Without wishing to be limited by theory, it is believed that these initial seeds comprise a complex of crystallized fat and Q10 whereupon the ubidecarenone starts to form the crystals according to the invention comprising a plurality of elongated parts, said elongated parts extending or protruding from a core region (i.e. needle-shaped crystals, or crystals having a "snow flake star form").

Optional step d):

The oil matrix of step c) is preferably cooled further (to room temperature) as part of the filling of the composition into softgel capsules.

In this step, the Q10 crystal is believed to be "coated" further with a layer of fat, which stabilizes the crystal in the solid form.

It was found that the desired needle-shaped crystals are formed and/or maintained independent of the cooling profile in step d). Further, it was found that the crystals are highly stable.

## EXAMPLES

### Example 1

A softgel capsule containing ubidecarenone crystals according to the invention was produced as follows: 74250 g (74.25 kg) soybean oil was loaded into a low speed mixer under continuous stirring with low speed mixer set at 10 rpm and at a pre-set temperature of 47°C. To this soybean oil was added 24750 g partly hydrogenated soybean oil with a melting point of 32°C, 5000 g partly hydrogenated soybean oil with a melting point of 41°C and 1000 g vitamin E under continuous stirring. An oil solution was formed.
25000g ubidecarenone powder was added to the solution under continuous stirring.

After addition of ubidecarenone powder the temperature was adjusted to 43°C. The solution was kept under constant stirring at 43°C for 60 minutes. The solution was visually checked and observed to be a clear solution without any visible floating crystals.

If the temperature is too low or there are visible crystals, then stirring and heating should continue.

The resulting solution was cooled and filled into softgel capsules each containing:

| | |
|---|---|
| Ubidecarenone | 100 mg |
| Soybean oil | 297 mg |
| Hydrogenated soybean oil with melting point 32°C | 79 mg |
| Hydrogenated soybean oil with melting point 41°C | 20 mg |
| Vitamin E 1 IU/mg | 4 mg |
| **Total** | **500 mg** |

Capsule shell:

| | |
|---|---|
| Gelatine | 110 - 148 mg |
| Glycerol | 47 - 64 mg |
| Colour | 2 mg |
| Purified water | 12 - 32 mg |
| **Grand total** | **approx. 700 mg** |

Cooling ubidecarenone according to this invention from the manufacturing temperature to room temperature at different rates was not found to have a significant effect on the ubidecarenone crystal formation. The primary question is to what extent ubidecarenone is dissolved at 37°C, prior to absorption.

From saturation point tests conducted over a period of 60 minute it was demonstrated that the ubidecarenone in the produced capsule formulations was completed dissolved at 37°C.

Similarly, NMR-tests, conducted with a heating-up period of 30 minutes, showed that the crystals were completely dissolved at 37°C (where detection limit is < 2%).

At 20°C, however, two methods of analysis showed a crystal percentage of about 18% and about 17%, respectively.

In phase-division testing, a "re-saturation" test showed that when the capsules were warmed up to 37°C after six minutes, small quantities of microscopic crystals could still be seen, leading to the conclusion that six minutes at 37°C is too short a period for a complete re-dissolution of the crystals. After 10 minutes (being the minimum time for disintegration of the capsules *in vivo*), visual detection of crystals was no longer possible, leading to the conclusion that the majority of the crystals were fully dissolved at the time of the disintegration of the capsules. After a heating-up period of 30 minutes at 37°C, the ubidecarenone needle-shaped crystals were completely dissolved (detection limit is < 2%).

It was further shown that the ubidecarenone needle-shaped crystal form according to the invention remains constant throughout storage of the product and is unaffected by changes in temperature during storage.

**Example 2** (Reference example)

Reference softgel capsules were produced by mixing the ingredients of Example 1 other than ubidecarenone at 47° followed by cooling to room temperature and adding ubidecarenone. Hereafter, the resulting dispersion was loaded into softgel capsules, each containing the same ingredients and amounts thereof as the softgel capsules according to Example 1.

The content of the softgel capsules produced according to Example 1 and the reference softgel capsules produced according to Example 2 were analysed by microscopy. The results confirmed the presence of ubidecarenone crystals (>99%) according to the invention in the softgel capsules produced according to Example 1. The reference capsule contained only rhomboid ubidecarenone.

When heating the softgel capsules in a water bath to a temperature of 37°C, it was further observed (by NMR) that the contents of the capsules according to Example 1 became liquid and that substantially all needle-shaped ubidecarenone crystals were solubilized.

This was not the case for the reference capsules, wherein the rhomboid crystals were observed.

**Example 3** (Reference example)

104000g soybean oil was loaded into a low speed mixer under continuous stirring with a low-speed mixer set at 10 rpm and at a pre-set temperature of 47°C. To this soybean oil was added 1000g vitamin E under continuous stirring.

25000g ubidecarenone powder with rhomboid ubidecarenone crystal structure was added to the blend under continuous stirring.

After addition of ubidecarenone powder, the temperature was adjusted to 43°C. The solution was kept under constant stirring at 43°C for 60 minutes. The solution was visually checked and observed to be a clear solution without any visible crystals. If the temperature is too low or there are visible crystals, then stirring and heating should continue. The resulting solution was filled into softgel capsules as described in Example 1.

The contents of the softgel capsules produced were analysed by microscopy and the absence of ubidecarenone crystals according to the present invention was confirmed.

**Example 4**

99750 g virgin olive oil (melting temperature approx. -6 °C) was loaded into a low-speed mixer under continuous stirring h a low-speed mixer set at 10 rpm and at a pre-set temperature of 47°C. To this virgin olive oil, 5250 g cocoa butter (melting temperature approx. 34°C) was added under continuous stirring.

25000 g ubidecarenone powder with rhomboid ubidecarenone crystal structure was added to the blend under continuous stirring. After addition of ubidecarenone powder, the temperature was adjusted to 43°C. The solution was kept under constant stirring at 43°C for 60 minutes. The solution was visually checked and observed to be a clear solution without any visible floating precipitate. If the temperature is too low or there are visible crystals, then stirring and heating should continue.

The resulting solution was filled into softgel capsules as described in Example 1.

The contents of the softgel capsules produced according to example 4 were analysed by microscopy. The results confirmed that the contents of ubidecarenone crystals in example 4 were similar to ubidecarenone crystals in the softgel capsules produced according to Example 1.

**Example 5**

8400 g virgin olive oil was loaded into a low-speed mixer under continuous stirring with a low-speed mixer set at 10 rpm and at a pre-set temperature of 70°C. To this virgin olive oil, 420 g hydrogenated soybean oil (melting temperature of . 65°) was added under continuous stirring. The hydrogenated soybean oil was melted and mixed with the virgin olive oil. When the blend became clear, it was mixed with 96180 g virgin olive oil at a temperature of 47°C.

25000 g ubidecarenone powder with rhomboid ubidecarenone crystal structure was added to the blend under continuous stirring. After addition of ubidecarenone powder, the temperature was adjusted to 43°C. The solution was kept under constant stirring at 43°C for 60 minutes. The solution was visually checked and observed to be a clear solution without any visible floating crystals. If the temperature is too low or there are visible crystals, then stirring and heating should continue.

The resulting solution was filled into softgel capsules as described in Example 1.

The contents of the softgel capsules produced according to example 5 were analysed by microscopy. The results confirmed that the contents of ubidecarenone crystals in Example 5 were similar to ubidecarenone crystals in the softgel capsules produced according to Example 1.

**Example 6**

*In vitro test*

The contents of three 100 mg ubidecarenone softgel capsules produced according to example 1 were emptied into an Eppendorf tube. As a reference, the contents of three 100 mg ubidecarenone softgel capsules produced according to Example 2 were emptied into an Eppendorf tube and analysed in parallel.

The two Eppendorf tubes were placed in a water bath at 31°C, and the temperature was then raised gradually to 38°C. When the temperature reached 37°C, the Eppendorf tubes were turned gently and carefully and repeatedly turned as the temperature increased to 38°C. At 38°C, the Eppendorf tubes were removed from the water bath and centrifuged at 14,000 rpm for 6 minutes at 37°C.

The Eppendorf tube containing the ubidecarenone crystals according to the invention produced only insignificant precipitate, whereas the reference tube containing rhomboid ubidecarenone crystals gave considerable amounts of crystallized precipitate that is not bioavailable.

**Example 7**

*In vivo test*

The purpose of this example was to compare the ubidecarenone crystalline structure, i.e. rhomboid versus needle-shaped crystals, in softgel capsules, and the influence thereof on the absorption of ubidecarenone in humans.

*Method*

1 softgel capsule produced according to Example 1 or a reference softgel capsule produced according to Example 2 was given to five healthy male volunteers aged 23 - 33 years in a randomised, double-blind, single-dose crossover trial with a one-week interval between treatments. A blood sample was obtained from each person in the morning and immediately after the single capsule was taken with water.

After treatment, the subjects fasted for 5 hours, after which a second blood sample was obtained. The procedure was repeated with random crossover one week later.

Serum was obtained from the blood samples, and was kept frozen until analysed for ubidecarenone. All samples were analyzed in one run by a standard HPLC method.

*Results*

Capsules according to Example 1:

**Serum ubidecarenone concentration (mg/l)**

| Test person | Baseline | 5 hour value | Difference |
|---|---|---|---|
| 1 | 0.92 | 1.02 | 0.10 |
| 2 | 0.91 | 0.95 | 0.04 |
| 3 | 0.54 | 0.57 | 0.03 |
| 4 | 0.67 | 0.73 | 0.06 |
| 5 | 0.81 | 0.91 | 0.10 |

Median (and average) increase from baseline = 0.06 (0.07).

Capsules according to Example 2:

**Serum ubidecarenone concentration (mg/l)**

| Test person | Baseline | 5 hour value | Difference |
|---|---|---|---|
| 1 | 1.15 | 1.17 | 0.02 |
| 2 | 0.82 | 0.86 | 0.04 |
| 3 | 0.58 | 0.59 | 0.01 |
| 4 | 0.73 | 0.65 | - 0.08 |
| 5 | 1.28 | 1.18 | - 0.10 |

Median (and average) increase from baseline = 0.01 (-0.02).

The increase in serum concentration of ubidecarenone from the single dose of capsules containing ubidecarenone according to the invention was in 5 cases out of 5 greater than or equal to the reference that contains rhomboid crystals of ubidecarenone:

| Test | Rhomboid | Cone-shaped | Difference |
|---|---|---|---|
| 1 | 0.02 | 0.10 | 0.08 |
| 2 | 0.04 | 0.04 | 0.00 |
| 3 | 0.01 | 0.03 | 0.02 |
| 4 | - 0.08 | 0.06 | 0.14 |
| 5 | - 0.10 | 0.10 | 0.20 |
| Median | 0.01 | 0.06 | 0.08 |

The median increase of plasma ubidecarenone found in this study with 5 fasting persons at 5 hours after a single dose of softgel capsule with 100 mg ubidecarenone was 0.06 mg/l for softgel capsules that contain ubidecarenone according to the invention, and 0.01 mg/l in the reference rhomboid formulation.

The study confirms a significant difference in absorption of the two forms (p=0.0625) in favor of the crystal form of ubidecarenone according to the invention. Hence, this example indicates an *in vivo* difference in the rate of absorption of ubidecarenone formulations between cone-shaped crystals and rhomboid crystals.

**Example 8** - **In vivo clinical trial**

*Study design*

A clinical trial was designed as a plasma Q10 concentration - time presentation above baseline of two ubidecarenone soft gelatine capsules that are identical except for the crystal structure of Q10. A cross-over design was employed, with a wash-out period of minimum 2 weeks between intakes of each formulation to avoid influence from prior test. Sequence of presentation of formulations is random.

*Methods and materials*

The formulations tested were ubidecarenone (Q10) capsules with a specified content of 100 mg ubiquinone each, manufactured by Pharma Nord. The capsules were identical except for the crystal structure of Q10 in the capsules. Formulation 1 (Batch POW597) was made according to example 1 of the present application and formulation 2 (KOJ786) was made according to example 2 (reference example) of the present application.
The concentration of ubiquinone in each preparation was analyzed and was found to be 99.12 mg per capsule in the product according to example 1 and 99.67 mg per capsule in product according to example 2.
The shape of ubiquinone crystals in the formulations was examined by microscope after production of capsules and the presence of star shaped crystals of ubiquinone in the product according to example 1 (POW597) was confirmed. The product according to example 2 (KOJ786) contained rhomboid crystals of ubiquinone.

*Subjects*

A group of healthy Spanish students aged 18 to 33 years (10 males and 4 females with a range of weight of between 52 and 86 kg) were included in the study. Signed informed consent was obtained from each subject prior to inclusion.

*Trials*

Subjects were given the softgel capsules containing Q10 as a single dose. Capsules in each step were distributed among subjects at random as a single dose. The treatment scheme for each subject was randomly chosen, and the composition of preparations given to each subject were unknown to the subjects and to the investigator, but could be traced by letter codes.
All tests were carried out during weekends with start at 8 a.m. on Saturday. Subjects were fasting overnight from Friday to Saturday. A wash out period of minimum 2 weeks between intakes of each formulation was used to avoid influence from prior test.
Prior to treatment, blood samples were obtained to determine the baseline. Soft gel capsules containing the ubiquinone formulations were then taken with a glass of water, where after subjects were provided with a breakfast consisting of milk products such as milk or yogurt together with a ham sandwich. In addition, fruit juices and water are available ad libitum. After four hours, a lunch consisting of meat, fish, salad and fresh fruits was served.
Most subjects consumed water but a few also drank red wine.

**Results:**

Blood samples were obtained at -1, 2, 4, 6, 8, 24 and 48 hours and analyzed for concentration of ubiquinone (Q10) in plasma (mg per liter).

**Table 1:** *Change in plasma ubidecarenone (Q10) concentration (mean, $\pm$SD and $\pm$SE) during the first 48 hours after ingestion of a single dose of a 100 mg soft gelatine capsule containing star-shaped crystals of ubidecarenone according to example 1.*

| Time Hours | -1 | 2 | 4 | 6 | 8 | 24 | 48 |
|---|---|---|---|---|---|---|---|
| Mean | 0,00 | 0,00 | 0,11 | 0,40 | 0,43 | 0,37 | 0,16 |
| SD | 0,00 | 0,07 | 0,13 | 0,36 | 0,25 | 0,22 | 0,15 |
| SE | 0,00 | 0,02 | 0,04 | 0,11 | 0,07 | 0,07 | 0,04 |

**Table 2:** *Change in plasma ubidecarenone (Q10) concentration (mean, $\pm$SD and $\pm$SE) during the first 48 hours after ingestion of a single dose of a 100 mg soft gelatine capsule containing rhomboid crystals of ubidecarenone according to Example 2.*

| Time Hours | -1 | 2 | 4 | 6 | 8 | 24 | 48 |
|---|---|---|---|---|---|---|---|
| Mean | 0.00 | -0,01 | -0,01 | 0,10 | 0,07 | 0,14 | 0,16 |

(continued)

| Time Hours | -1 | 2 | 4 | 6 | 8 | 24 | 48 |
|---|---|---|---|---|---|---|---|
| SD | 0.00 | 0,08 | 0,11 | 0,18 | 0,15 | 0,14 | 0,13 |
| SE | 0,00 | 0,02 | 0,03 | 0,05 | 0,04 | 0,04 | 0,03 |

As seen in Tables 1 and 2, the concentration (mean±SE) of ubidecarenone in plasma in mgQ10/L increases after intake of both capsules.

The natural baseline levels were 0.34 mg ubidecarenone (Q10)/L. As seen from Table 1, the relative Q10-plasma levels increases 2 hours after intake of the soft-gelatine capsules and reaches the primary peak 8 hours after intake.

Subject treated with the product (POW597) containing star-shaped crystals according to the invention reach the primary peak level of 0.77 mgQ10/L, which is 0.43 mgQ10/L higher than the initial level. The concentration of ubidecarenone in plasma thereafter decreases but is maintained after 24 hours at a level of 86% relative increase compared to the maximum concentration after 8 hours.

As seen in table 2 the concentration (mean±SE) of ubidecarenone in plasma in mgQ10/L after intake of reference product (KOJ786) increases from a baseline level of 0.37 mg Q10/L. The relative Q10-plasma levels increases 4 hours after intake of the soft-gelatine capsules and reaches the primary peak after 6 hours, where the level is 0.10 mg/l higher than the initial level, thus reaching 0.47 mgQ10/L. Concentration of ubidecarenone in plasma is sustained throughout the remaining study period.

The results show that the star-shaped crystals according to the invention raises the concentration of ubidecarenone in plasma to concentrations that are considerably higher from 4 and 24 hours compared with product that contains rhomboid crystals. T-test shows a p-value of ≤0.01 in al data-points in this interval (t = 4, 6, 8 and 24h).

The 6 - 8 hours absolute Cmax and ΔCmax (increase) for the product with star-shaped crystals are respectively 0.77 and 0.43 mg Q10/L. The 6 - 8 hours absolute Cmax and ΔCmax (increase) for the product with rhomboid crystals are respectively 0.47 and 0.10 mg Q10/l. Thus, the absolute Cmax differs by a factor of 0.77:0.47 = 1.6. The relative Cmax differs by a factor of 0.43:0.10= 4.3. T-test shows a p-value of 0.001 for ΔC8 hours.

The area under the curve (AUC) as an expression for the absorbed fraction from 0 hours to 48 hours has been calculated to be 7.1 mgQ10/L/day for the capsule containing Q10 with star-shaped crystal structure according to the invention, versus 2.8 mgQ10/L/day for the capsule with the reference rhomboid crystals. The relative difference of absorption thus differs with a factor of 7.1:2.8=2.5. Thus, absorption over the first 48 h differs by a factor of 7.1:2.8 = 2.5.

The AUC0-24h is 7.8 mgQ10/L/day for the capsules containing Q10 with star-shaped crystal structure according to the invention and 1.9 mgQ10/L/day for the reference rhomboid crystals. Thus, absorption over the first 24 hours differs by a factor of 7.8/1.9 = 4.1. The difference in absorption between the two formulations is greater during the first 24 h. The in vivo difference between the old and the new crystal structure in the capsules is significant and in favor of the new star shaped crystal structure.

The results clearly show the advantage in human in vivo absorption of the star-shaped crystal structure according to the invention compared to the conventional available rhomboid crystal structure of ubidecarenone raw material.

**Claims**

1. Crystal form of ubidecarenone, wherein the ratio of the crystal surface area measured in $\mu m^2$ per volume measured in $\mu m^3$ of the ubidecarenone crystals is greater than 2 and wherein the crystal form of ubidecarenone comprises a plurality of needle-shaped or cone-shaped elongated parts having an average length of at least 10 $\mu m$ and a diameter at the base of less than 1$\mu m$, said elongated parts extending or protruding from a core region.

2. Crystal form of ubidecarenone according to claim 1, wherein the needle-shaped or cone-shaped elongated parts have an average length of 10-100 $\mu m$ and an average diameter at the base of 0.01 - 1$\mu m$.

3. A method of preparing a crystal form of ubidecarenone according to any of claims 1 or 2, comprising the steps of:

    a) providing fat or oil composition comprising one or more low-temperature melting triglycerides and one or more high-temperature melting triglycerides, said low-temperature melting triglycerides having melting points that are at least 10°C lower than the melting temperature of the high-temperature melting triglycerides,

b) solubilizing ubidecarenone in the composition of step a),

c) lowering the temperature of the composition of step b) to a temperature below the temperature at which the amount of Q10 solubilizes in the composition, thereby providing an oversaturated composition, for a period sufficient to allow formation of Q10 crystals according to claim 1, and

d) optionally, cooling the composition of step c) to room temperature.

4. Method according to claim 3, wherein the composition of step a) is heated to a temperature that is above the melting temperature of the one or more high-temperature melting triglycerides.

5. Method according to claim 3 or 4, wherein the composition of step b) is saturated with ubidecarenone.

6. Method according to any of claims 3- 5, wherein the composition of step a) comprises a low-temperature melting triglyceride having a melting point of below 15°C and a high- temperature melting triglyceride having a melting point of at least 30°C.

7. Method according to claim 6, wherein at least one of the high-temperature melting triglycerides added in step a) is selected among the group comprising hydrogenated or partially hydrogenated triglycerides as well as natural fat or oils with a high concentration of saturated fat such as cocoa butter, coconut oil, hydrogenated soybean oil, palm kernel oil, palm oil or shea fat.

8. Method according to any of claims 6 - 7, wherein the high-temperature melting triglycerides added in step a) are a combination of at least two different triglycerides selected among the group comprising hydrogenated or partially hydrogenated triglycerides as well as natural triglycerides containing a high amount of saturated fat, such as cocoa butter, coconut oil, palm kernel oil, palm oil and shea fat, and step b) is performed at a temperature at or above the melting temperature of ubidecarenone.

9. A composition comprising crystals of ubidecarenone according to any of claims 1 or 2, and one or more triglycerides.

10. Composition according to claim 9, wherein the ratio of fat and/or oil to ubidecarenone per weight is between 2:1 and 20:1, preferably 4:1.

11. Composition according to any one of claims 9-10 for use as a medicine.

12. Use of a composition according to any one of claims 9-10 as a food or food supplement.

13. Ubidecarenone according to any of claims 1 or 2 for use as a medicine.

14. Use of a ubidecarenone according to any of claims 1 or 2 as a food or food supplement.

**Patentansprüche**

1. Kristallform von Ubidecarenon, wobei das Verhältnis der Kristalloberfläche, gemessen in $\mu m^2$, pro Volumen, gemessen in $\mu m^3$, der Ubidecarenonkristalle, größer als 2 ist und wobei die Kristallform von Ubidecarenon eine Vielzahl von nadelförmigen oder kegelförmigen länglichen Teilen mit einer durchschnittlichen Länge von mindestens 10 $\mu m$ und einem Durchmesser an der Basis von weniger als 1 $\mu m$ umfasst, wobei sich die länglichen Teile erstrecken oder vorstehen von einem Kernbereich.

2. Kristallform von Ubidecarenon nach Anspruch 1, wobei die nadelförmigen oder kegelförmigen länglichen Teile eine durchschnittliche Länge von 10-100 $\mu m$ und einen durchschnittlichen Durchmesser an der Basis von 0,01 - 1 $\mu m$ aufweisen.

3. Verfahren zur Herstellung einer Kristallform von Ubidecarenon nach einem der Ansprüche 1 oder 2, umfassend die Schritte:

a) Bereitstellen einer Fett- oder Ölzusammensetzung, umfassend ein oder mehrere niedrigschmelzende Triglyceride und ein oder mehrere hochschmelzende Triglyceride, wobei die niedrigschmelzenden Triglyceride Schmelzpunkte aufweisen, die mindestens 10°C niedriger sind als die Schmelztemperatur der hochschmelzenden Triglyceride,

b) Löslichmachen von Ubidecarenon in der Zusammensetzung von Schritt a),

c) Senken der Temperatur der Zusammensetzung von Schritt b) auf eine Temperatur unterhalb der Temperatur, bei der die Menge an Q10 in der Zusammensetzung löslich ist, wodurch eine übersättigte Zusammensetzung bereitgestellt wird, für einen Zeitraum, der ausreicht, um die Bildung von Q10-Kristallen nach Anspruch 1 zu ermöglichen, und

d) gegebenenfalls Kühlen der Zusammensetzung von Schritt c) auf Raumtemperatur.

**4.** Verfahren nach Anspruch 3, wobei die Zusammensetzung von Schritt a) auf eine Temperatur erwärmt wird, die über der Schmelztemperatur des einen oder der mehreren hochschmelzenden Triglyceride liegt.

**5.** Verfahren nach Anspruch 3 oder 4, wobei die Zusammensetzung von Schritt b) mit Ubidecarenon gesättigt ist.

**6.** Verfahren nach einem der Ansprüche 3-5, wobei die Zusammensetzung von Schritt a) ein niedrigschmelzendes Triglycerid mit einem Schmelzpunkt von unter 15°C und ein hochschmelzendes Triglycerid mit einem Schmelzpunkt von mindestens 30°C umfasst.

**7.** Verfahren nach Anspruch 6, wobei mindestens eines der in Schritt a) zugesetzten hochschmelzenden Triglyceride ausgewählt ist aus der Gruppe, umfassend hydrierte oder teilweise hydrierte Triglyceride sowie natürliches Fett oder Öle mit einer hohen Konzentration an gesättigtem Fett, wie Kakaobutter, Kokosöl, hydriertes Sojaöl, Palmkernöl, Palmöl oder Sheafett.

**8.** Verfahren nach einem der Ansprüche 6-7, wobei die in Schritt a) zugegebenen hochschmelzenden Triglyceride eine Kombination aus mindestens zwei verschiedenen Triglyceriden sind, ausgewählt aus der Gruppe, umfassend hydrierte oder teilweise hydrierte Triglyceride sowie natürliche Triglyceride mit einer hohen Menge an gesättigtem Fett, wie Kakaobutter, Kokosöl, Palmkernöl, Palmöl und Sheafett, und Schritt b) bei einer Temperatur bei oder über der Schmelztemperatur von Ubidecarenon durchgeführt wird.

**9.** Zusammensetzung, umfassend Kristalle von Ubidecarenon nach einem der Ansprüche 1 oder 2 und ein oder mehrere Triglyceride.

**10.** Zusammensetzung nach Anspruch 9, wobei das Verhältnis von Fett und/oder Öl zu Ubidecarenon pro Gewicht zwischen 2:1 und 20:1, vorzugsweise 4:1, liegt.

**11.** Zusammensetzung nach einem der Ansprüche 9-10 zur Verwendung als Medikament.

**12.** Verwendung einer Zusammensetzung nach einem der Ansprüche 9-10 als Nahrungsmittel oder Nahrungsergänzungsmittel.

**13.** Ubidecarenon nach einem der Ansprüche 1 oder 2 zur Verwendung als Medikament.

**14.** Verwendung eines Ubidecarenons nach einem der Ansprüche 1 oder 2 als Nahrungsmittel oder Nahrungsergänzungsmittel.

## Revendications

**1.** Forme cristalline de l'ubidécarénone, dans laquelle le rapport de surface cristalline mesurée en $\mu m^2$ par volume mesuré en $\mu m^3$ des cristaux d'ubidécarénone est supérieur à 2 et dans laquelle la forme cristalline de l'ubidécarénone comprend une pluralité de parties allongées en forme d'aiguille ou de cône ayant une longueur moyenne d'au moins 10 $\mu m$ et un diamètre à la base inférieur à 1 $\mu m$, lesdites parties allongées s'étendant ou faisant saillie depuis une région centrale.

**2.** Forme cristalline de l'ubidécarénone selon la revendication 1, dans laquelle les parties allongées en forme d'aiguille ou en forme de cône ont une longueur moyenne de 10 à 100 $\mu m$ et un diamètre moyen à la base de 0,01 à 1 $\mu m$.

**3.** Procédé de préparation d'une forme cristalline d'ubidécarénone selon l'une quelconque des revendications 1 ou 2, comprenant les étapes de :

a) fournir une composition de graisse ou d'huile comprenant un ou plusieurs triglycérides à fusion à basse température et un ou plusieurs triglycérides à fusion à haute température, lesdits triglycérides à fusion à basse température ayant des points de fusion qui sont inférieurs d'au moins 10 °C à la température de fusion des triglycérides à fusion à haute température ;

b) solubiliser l'ubidécarénone dans la composition de l'étape a) ;

c) abaisser la température de la composition de l'étape b) jusqu'à une température inférieure à la température à laquelle la quantité de Q10 se solubilise dans la composition, fournissant ainsi une composition sursaturée, pendant une durée suffisante pour permettre la formation de cristaux de Q10 selon la revendication 1, et

d) optionnellement, refroidir la composition de l'étape c) à la température ambiante.

4. Procédé selon la revendication 3, dans lequel la composition de l'étape a) est chauffée à une température qui est supérieure à la température de fusion du ou des triglycérides à fusion à haute température.

5. Procédé selon la revendication 3 ou 4, dans lequel la composition de l'étape b) est saturée en ubidécarénone.

6. Procédé selon l'une quelconque des revendications 3 à 5, dans lequel la composition de l'étape a) comprend un triglycéride à basse température de fusion ayant un point de fusion inférieur à 15 °C et un triglycéride à fusion à haute température ayant un point de fusion d'au moins 30 °C.

7. Procédé selon la revendication 6, dans lequel l'un au moins des triglycérides à fusion à haute température ajoutés à l'étape a) est choisi dans le groupe comprenant les triglycérides hydrogénés ou partiellement hydrogénés ainsi que des graisses ou huiles naturelles à forte teneur en graisses saturées comme le beurre de cacao, l'huile de noix de coco, l'huile de soja hydrogénée, l'huile de noix de palme, l'huile de palme ou la graisse de karité.

8. Procédé selon l'une quelconque des revendications 6 à 7, dans lequel les triglycérides à fusion à haute température ajoutés à l'étape a) sont une combinaison d'au moins deux triglycérides différents choisis dans le groupe comprenant les triglycérides hydrogénés ou partiellement hydrogénés ainsi que des triglycérides naturels contenant une quantité élevée de graisse saturée, comme le beurre de cacao, l'huile de noix de coco, l'huile de noix de palme, l'huile de palme et la graisse de karité, et l'étape b) est réalisée à une température égale ou supérieure à la température de fusion de l'ubidécarénone.

9. Composition comprenant des cristaux d'ubidécarénone selon l'une quelconque des revendications 1 ou 2, et un ou plusieurs triglycérides.

10. Composition selon la revendication 9, dans laquelle le ratio de graisse et/ou d'huile par rapport à l'ubidécarénone par poids est compris entre 2:1 et 20:1, de préférence 4:1.

11. Composition selon l'une quelconque des revendications 9 à 10 pour une utilisation en tant que médicament.

12. Utilisation d'une composition selon l'une quelconque des revendications 9 à 10 comme aliment ou complément alimentaire.

13. Ubidécarénone selon l'une quelconque des revendications 1 ou 2 pour une utilisation en tant que médicament.

14. Utilisation d'une ubidécarénone selon l'une quelconque des revendications 1 ou 2 en tant qu'aliment ou complément alimentaire.

**FIG. 1**

**FIG. 2**

**FIG. 3**

**FIG. 4**

Q10 Absorption Comparison

**FIG. 5**

**EP 3 191 444 B1**

**REFERENCES CITED IN THE DESCRIPTION**

**Patent documents cited in the description**

- CN 103819832 A
- CN 103819326 A
- US 4220719 A1
- JP S516106597 A